# EUROPÄISCHE PATENTSCHRIFT

(11) **EP 3 747 081 B1**
(45) Veröffentlichungstag und Bekanntmachung des Hinweises auf die Patenterteilung: **21.09.2022**
(21) Anmeldenummer: 19701194.3
(22) Anmeldetag: 17.01.2019
(51) Int. Cl.: H01P 1/06, A61B 6/00

(54) **VORRICHTUNG ZUR ÜBERTRAGUNG EINES SIGNALS MITTELS WELLENLEITERN**
DEVICE FOR TRANSFERRING A SIGNAL BY MEANS OF WAVEGUIDES
DISPOSITIF DE TRANSMISSION D'UN SIGNAL PAR GUIDE D'ONDE

(30) Priorität: 01.02.2018 DE 102018201510
(43) Veröffentlichungstag der Anmeldung: 09.12.2020
(73) Patentinhaber: Robert Bosch GmbH, 70442 Stuttgart (DE)
(72) Erfinder: BRECHTER, Ludwig, 70469 Stuttgart (DE); WORMS, Kai, 74239 Hardthausen Am Kocher (DE)
(86) Internationale Anmeldenummer: PCT/EP2019/051149
(87) Internationale Veröffentlichungsnummer: WO 2019/149535

(56) Entgegenhaltungen:
- DE-A1- 19 533 820
- DE-A1- 19 543 559
- DE-A1-102011 050 588
- JP-A- S63 220 401
- US-B1- 6 181 766

## Beschreibung

Die vorliegende Erfindung betrifft eine Vorrichtung zur Übertragung eines Signals mittels Wellenleitern in rotierenden Systemen, umfassend wenigstens einen Sende-Wellenleiter und wenigstens einen Empfangs-Wellenleiter, wobei der wenigstens eine Empfangs-Wellenleiter und der wenigstens eine Sende-Wellenleiter in mehrere radial verteilte Segmente unterteilt sind.

### Stand der Technik

Datenübertragungen zwischen einem kontinuierlich rotierenden und einem stationären Bereich werden in verschiedensten Anwendungsfällen benötigt. Als Beispiele sind Windenergieanlagen, Computertomographen, Roboter und rotierende Laser-Radare zu nennen. Für Datenraten ab einigen Mbit/s sind dabei verschleißfreie, kontaktlose Verfahren den klassische Schleifringe einsetzenden Verfahren überlegen. Ein mögliches kontaktloses Verfahren basiert auf der Datenübertragung im Nahfeld von sich gegenüberliegenden Wellenleitern.

Das Prinzip eines solchen Übertragungsverfahrens ist beispielsweise in der DE 195 43 558 B4 beschrieben. Dabei ist eine Signalquelle an ein Sender-Wellenleiterpaar angeschlossen, wobei ein zu übertragendes Signal elektromagnetisch auf ein zweites, parallel verlaufendes Wellenleiterpaar überkoppelt, welches somit als Empfänger dient. Das Wellenleiterpaar des Empfängers ist typischerweise kürzer als das des Senders. Um eine Übertragung zu erreichen, die gegenüber elektromagnetischer Strahlung unempfindlich ist und zudem wenig elektromagnetische Strahlung im Fernfeld emittiert, wird typischerweise ein symmetrischer Aufbau von Sender und Empfänger gewählt. Dies bedeutet, dass der Hinweg des Stroms zum Empfänger und dessen Rückweg über gleich ausgeführte Strukturen führt. Demnach müssen die beiden Wellenleiter des Wellenleiterpaars zum Senden und die beiden Wellenleiter des Wellenleiterpaars zum Empfangen jeweils die gleiche Fläche aufweisen.

Ferner ist aus der DE 195 43 559 A1 ein Signalverlauf eines WellenleiterKopplers bekannt, welcher an verschiedenen Stellen während der Übertragung gezeigt wird. Dementsprechend weist ein Eingangs-Datensignal eine Rechteckform auf, wobei mittels eines Komparators die Flankensteilheit dieses Signals erhöht wird. Mithilfe der Gleichung *λ*ₘᵢₙ=*c2πτ* kann dabei die geringste relevante Wellenlänge *λ*ₘᵢₙ anhand der Anstiegszeit *τ* der Flanke und der Ausbreitungsgeschwindigkeit c der Welle abgeschätzt werden. Durch die elektromagnetische Kopplung vom Sende-Wellenleiterpaar zum Empfangs-Wellenleiterpaar erfolgt eine Hochpassfilterung des Eingangssignals, welche zu einem Ausgangssignal führt, das aus Pulsen besteht, die im Bereich der Signalflanken des Eingangssignals auftreten. Nach Durchlaufen des Wellenleiterkopplers wird durch einen Integrator oder einen Komparator mit Hysterese das rechteckförmige Signal wiederhergestellt.

Des Weiteren ist aus der DE 195 33 820 B4 ein auf Wellenleitern basierender Drehübertrager bekannt, bei welchem eine Datenübertragung für jeden Winkel des Empfängers zum Sender funktioniert. Um durch ein zu übertragendes Signal hervorgerufene Reflexionen zu vermeiden, weisen die Wellenleiter einen definierten Wellenwiderstand auf, auf welchen der Drehübertrager mittels Anpassungswiderständen eingestellt wird. Ferner werden Reflexionen in den Empfangsantennen vermieden, indem diese wesentlich kürzer ausgeführt werden als die kleinste vorkommende Wellenlänge *λ*ₘᵢₙ.

Gemäß dem Stand der Technik ist eine besonders einfache Aufbauart eines Systems zur Drehübertragung in Form von zwei gegenüberliegenden Leiterplatten gegeben, wobei verwendete Wellenleiter in der Form von Mikrostreifenleitungen ausgeführt werden.

Als nachteilig ist bei den vorgenannten aus dem Stand der Technik bekannten Lösungen anzusehen, dass derzeitige Wellenleiter-Drehübertrager über einen Empfangs-Wellenleiter verfügt, der wesentlich kürzer ist als die als die kleinste im Signal vorkommende Wellenlänge *λ*ₘᵢₙ. Dadurch ist der Empfangs-Wellenleiter bei hohen Übertragungsraten deutlich kleiner als der Umfang des Sende-Wellenleiters. Aufgrund der hierdurch resultierenden geringen Koppelfläche ist die Koppeleffizienz so gering, dass am Sender und Empfänger zusätzlich Verstärker benötigt werden. Dies erhöht zum einen die Abstrahlung des Senders und zum anderen die Kosten und den Energieverbrauch des Wellenleiter-Drehübertragers.

Ein weiterer Nachteil einer solchen Anordnung besteht darin, dass die empfangene Signalamplitude abhängig von dem Abstand zwischen Empfangs-Wellenleiter und Sende-Wellenleiter ist. Wird das System in Form von zwei gegenüberliegenden Leiterplatten aufgebaut, führt bereits eine leichte Verkippung der Leiterplatten zueinander oder eine Unebenheit einer Leiterplatte dazu, dass die Signalamplitude während einer Umdrehung derart stark variiert, dass es abhängig vom Drehwinkel zeitweise zu Fehlern in der Datenübertragung kommt.

Anhand von Rückkoppelmechanismen kann der Empfänger die variierende Signalamplitude ausgleichen. Dies ist beispielsweise in der US 2004/0116099 A1 beschrieben, wobei entsprechend einer bevorzugten Methode der Einsatz eines Verstärkers mit einstellbarer Verstärkung vorgesehen ist. Ein solcher Verstärker ist jedoch kostenintensiv und benötigt viel Energie.

JP S63 220401 A, DE 10 2011 050588 A1 und US 6 181 766 B1 offenbaren Vorrichtungen zur Übertragung eines Signals mittels Wellenleitern in rotierenden Systemen, aber nur mit einem Sende-Wellenleiter.

DE 195 33 820 A1 offenbart eine ähnliche Vorrichtung mit zwei Sende-Wellenleitern. Der Empfangs-Wellenleiter besteht aber aus einem einzelnen Segment.

### Offenbarung der Erfindung

Erfindungsgemäß wird daher eine Vorrichtung zur Übertragung eines Signals mittels Wellenleitern in rotierenden Systemen zur Verfügung gestellt, umfassend die Merkmale von Anspruch 1.

Die Wellenleiter können dabei vorzugsweise gekoppelte Wellenleiter sein, wobei besonders bevorzugt gekoppelte Wellenleiter in einem inhomogenen Dielektrikum zum Einsatz kommen.

### Vorteile der Erfindung

Der erfindungsgemäß erhaltene Wellenleiter-Drehübertragers hat den Vorteil, dass er weitgehend unempfindlich gegenüber Unebenheiten und Verkippung ist. Dies wird dadurch erreicht, dass der Empfangs-Wellenleiter in mehrere, radial verteilte Segmente unterteilt wird, deren Empfangssignale aufaddiert werden.

Durch Addition der Signale aller Empfangs-Wellenleiter wird die Amplitude des Ergebnisses unabhängig von der Winkelposition, wodurch eine umständliche Nachregelung der Signalamplitude am Empfänger überflüssig wird.

Gemäß einer bevorzugten Ausgestaltung der Erfindung ist vorgesehen, dass sich die Abgriffe der radial verteilten Segmente des wenigstens einen Empfangs-Wellenleiters gegenüberliegen. Dadurch wird es ermöglicht, dass die Empfangs-Amplitude eines Segmentes, welche aufgrund von Verkippung oder einer Unebenheit reduziert wird, durch eine andere Empfangs-Amplitude des gegenüberliegenden Segmentes, welche dagegen hoch ist, ausgeglichen werden kann.

Vorteilhafterweise umfasst die Vorrichtung Mittel zur Einkopplung des zu übertragenden Signals mit positiver Polarität in ein erstes Segment des wenigstens einen Sende-Wellenleiters und zur Einkopplung des zu übertragenden Signals mit negativer Polarität in ein zweites Segment des wenigstens einen Sende-Wellenleiters. Dabei ist vorzugsweise vorgesehen, dass als Mittel zur Einkopplung des zu übertragenden Signals ein Komparator vorgesehen ist, welcher einen nichtinvertierenden und einen invertierenden Ausgang aufweist. Dadurch wird erreicht, dass der nichtinvertierende Ausgang ein rechteckförmiges Eingangssignal mit positiver Polarität in einen ersten Sende-Wellenleiter einkoppelt und der invertierende Ausgang das entsprechende Signal mit negativer Polarität in einen zweiten Sende-Wellenleiter einkoppelt, sodass beide Signale entlang der Wellenleiter während der Laufzeit in die Empfangs-Wellenleiter überkoppeln können.

Bevorzugter Weise ist zur Terminierung eines freibleibenden Endes der radial verteilten Segmente des wenigstens einen Sende-Wellenleiters und/oder des wenigstens einen Empfangs-Wellenleiters jeweils ein Widerstand vorgesehen. Auf diese Weise werden insbesondere die Empfangs-Wellenleiter reflexionsfrei abgeschlossen und können wesentlich länger sein als die minimal im Signal vorkommende Wellenlänge *λ*ₘᵢₙ, welche als geringste relevante Wellenlänge mittels *λ*ₘᵢₙ= *c*2π*τ* berechnet werden kann. Dies ist dadurch begründet, dass bei fehlangepassten Wellenleitern es bereits dann zu störenden Reflexionen kommen kann, wenn deren Länge größer als etwa 1/10 der maximal im Signal vorkommenden Wellenlänge ist. Die Wellenleiter bei einer vorliegenden kontaktlosen Datenübertragung sind nicht länger als die geringste Wellenlänge, sondern haben eine Länge von etwa 1/5 der geringsten relevanten Wellenlänge, so dass diese Wellenleiter nicht mehr wesentlich kürzer und niemals länger als die geringste Wellenlänge sind. Längere Wellenleiter würden hingegen zu einem Übersprechen zweier benachbarter Bits führen.

Gemäß einer Ausführungsform der Erfindung ist als Mittel zur Addition ein resistiver Koppler vorgesehen. Dies kann beispielsweise ein resistiver 50/50-Koppler sein, durch welchen unabhängig vom Winkel einer Verdrehung des Senders zum Empfänger ein Signal mit einer hohen Stabilität erhalten werden kann, indem die empfangenen Signale aller Segmente des Empfangswellenleiters addiert werden.

Vorteilhafterweise umfasst die Vorrichtung ferner Mittel zur Rekonstruktion des durch Addition erhaltenen Signals. Dies hat den Vorteil, dass das übertragene Signal, welches am Empfänger pulsförmig vorliegt, in das ursprüngliche rechteckförmige Signal rekonstruiert werden kann. Dazu ist als Mittel zur Rekonstruktion vorzugsweise ein Integrator oder ein Komparator mit Hysterese vorgesehen.

Gemäß einer bevorzugten Ausführungsform der Erfindung sind als Segmente für den wenigstens einen Sende-Wellenleiter und/oder für den wenigstens einen Empfangs-Wellenleiter Halbringe vorgesehen. Dadurch werden bei einem vergleichsweise geringen konstruktiven Aufwand dennoch die erfindungsgemäß gegebenen Vorteile erzielt.

Weiterhin sind alternativ als Segmente für den wenigstens einen Sende-Wellenleiter und/oder für den wenigstens einen Empfangs-Wellenleiter verkürzte Empfangsringe mit gegenüberliegenden Auskoppelpunkten vorgesehen. Dies hat den Vorteil, dass auf diese Weise geringere Signallaufzeiten erzielt werden können und dadurch höhere Bitraten ermöglicht werden.

Vorteilhafterweise ist die Länge der Segmente des wenigstens einen Empfangs-Wellenleiters größer als die minimal im zu übertragenden Signal vorkommende Wellenlänge. Dadurch kann erreicht werden, dass der Empfangs-Wellenleiter deutlich größer als bei bisher aus dem Stand der Technik bekannten Realisierungen ausgebildet werden kann, was zu einer höheren Koppelfläche und damit zu einer höheren Koppeleffizienz führt.

Weiter ermöglicht die erfindungsgemäße Vorrichtung, dass der wenigstens eine Sende-Wellenleiter an einem Rotor und der wenigstens eine Empfangs-Wellenleiter an einem Stator vorgesehen ist.

Die erfindungsgemäße Vorrichtung umfasst Mittel zur Aufteilung des zu übertragenden Signals für einen ersten Sende-Wellenleiter und für einen zweiten Sende-Wellenleiter umfasst. Auf diese Weise kann ein Wellenleiter-Drehübertrager in symmetrischer Ausführung erhalten werden, bei welchem ein Signal beispielsweise auf zwei Komparator-Eingänge aufgeteilt wird, wobei ein Komparator das Signal in das linke Wellenleiterpaar einkoppelt und der andere Komparator das Signal in das rechte Wellenleiterpaar einkoppelt.

Vorteilhafterweise umfasst die Vorrichtung in einer derartigen Ausführungsform einen ersten Empfangs-Wellenleiter und einen zweiten Empfangs-Wellenleiter, wobei der erste Empfangs-Wellenleiter zur Erfassung des mittels des ersten Sende-Wellenleiters übertragenen Signals und der zweite Empfangs-Wellenleiter zur Erfassung des mittels des zweiten Sende-Wellenleiters übertragenen Signals vorgesehen sind. Dadurch können auf Empfängerseite Signale erhalten werden, die durch Addition ein symmetrisches Signal bilden, aus dem der ursprüngliche rechteckförmige Datenstrom wiederhergestellt werden kann.

Vorteilhafte Weiterbildungen der Erfindung sind in den Unteransprüchen angegeben und in der Beschreibung beschrieben.

### Zeichnungen

Ausführungsbeispiele der Erfindung werden anhand der Zeichnungen und der nachfolgenden Beschreibung näher erläutert. Es zeigen:
Figur 1 eine Vorrichtung gemäß einem ersten Ausführungsbeispiel, und
Figur 2 eine erfindungsgemäße Vorrichtung in symmetrischer Ausführung gemäß einem zweiten Ausführungsbeispiel.

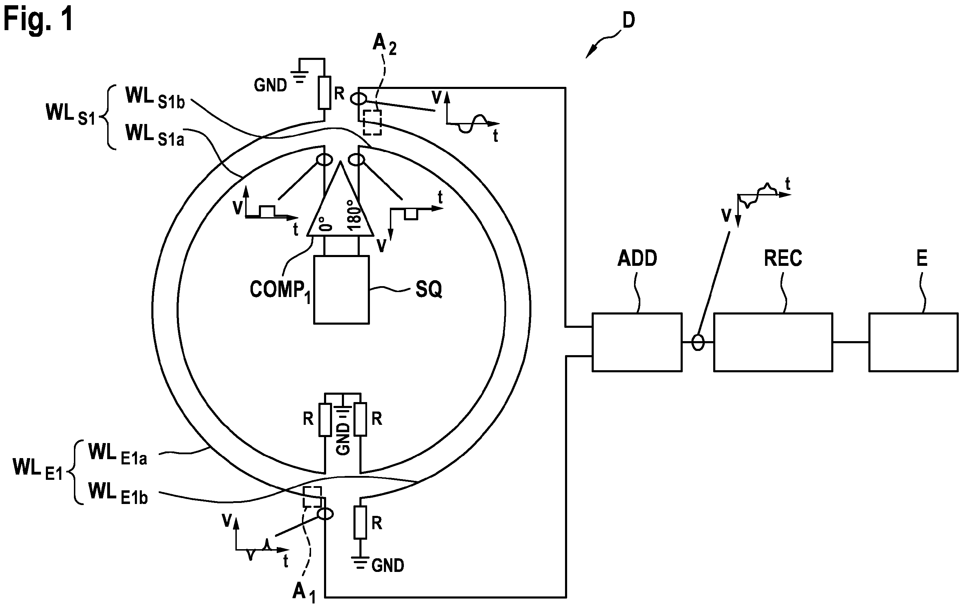

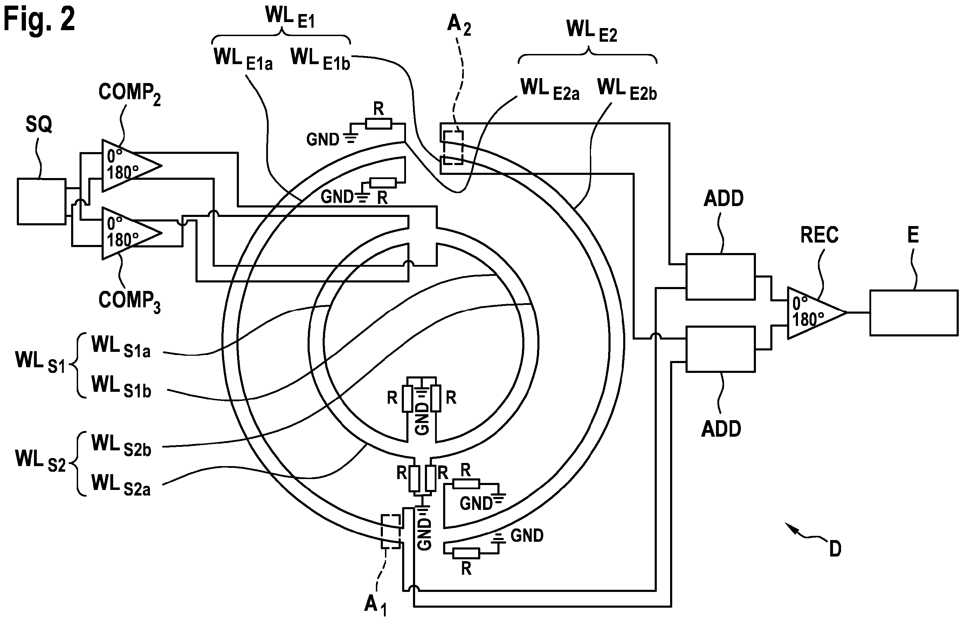

Das Ausführungsbeispiel von der Figur 1 hat nicht alle beanspruchten Merkmale, aber ist kompatibel mit dem erfindungsgemäßen Ausführungsbeispiel der Figur 2.

### Ausführungsformen der Erfindung

In Figur 1 ist eine Vorrichtung in Form eines Wellenleiter-Drehübertragers D gemäß einem ersten Ausführungsbeispiel gezeigt, welche weitgehend unempfindlich gegenüber Unebenheiten und Verkippung ist. Der Empfangs-Wellenleiter WL_{E1} ist zu diesem Zweck in mehrere, radial verteilte Segmente WL_{E1a}, WL_{E1b} unterteilt, deren Empfangssignale mittels eines Addierers ADD aufaddiert werden.

Ist beispielsweise die Empfangs-Amplitude eines Segmentes WL_{E1a} aufgrund einer Verkippung oder einer Unebenheit reduziert, so ist die Empfangs-Amplitude des gegenüberliegenden Segmentes W_{LE1b} hoch. Durch Addition der Signale aller Segmente WL_{E1a}, WL_{E1b} des Empfangs-Wellenleiters WL_{E1} wird die Amplitude des Ergebnisses unabhängig von der Winkelposition gemacht, was eine umständliche Nachregelung der Signalamplitude am Empfänger E überflüssig macht.

Vorliegend sind entsprechend Figur 1 die Segmente WL_{S1a}, WL_{S1b} des Sende-Wellenleiters WL_{S1} und die Segmente WL_{E1a}, WL_{E1b} des Empfangs-Wellenleiters WL_{E1} als Halbringe ausgeführt. Die Signale, die an einer Seite der Sende-Halbringe WL_{S1a}, WL_{S1b} eingekoppelt werden, sind zueinander invertiert. An der anderen Seite der Sende-Halbringe WL_{S1a}, WL_{S1b} befinden sich Terminierungs-Widerstände R.

Die Abgriffe A₁, A₂ der Segmente WL_{E1a}, WL_{E1b} des Empfangs-Wellenleiters WL_{E1} befinden sich an gegenüberliegenden Positionen, wobei das freibleibende Ende mit jeweils einem Widerstand R terminiert wird. Somit sind die Segmente WL_{E1a}, WL_{E1b} des Empfangs-Wellenleiters WL_{E1} reflexionsfrei abgeschlossen und können wesentlich länger sein als die minimal im Signal vorkommende Wellenlänge *λ*ₘᵢₙ. Durch diesen Aufbau wird die Fläche maximiert, über welche die elektromagnetische Welle des Sende-Wellenleiters WL_{S1} auf die des Empfangs-Wellenleiters WL_{E1} überkoppeln kann. Dies führt zu einer hohen Kopplungs-Effizienz, welche eine Vor- oder Nachverstärkung des Signals überflüssig macht. Gleichzeitig funktioniert diese Anordnung selbst bei hohen Datenraten von typischerweise 1Gbit/s.

Wie in Figur 1 weiter gezeigt ist, befindet sich hinter der Signalquelle SQ ein Komparator COMP₁, der die Flankensteilheit des rechteckförmigen Signals erhöht. Der nichtinvertierende Ausgang 0° koppelt ein rechteckförmiges Signal mit positiver Polarität in das linke Segment WL_{S1a} des Sende-Wellenleiters WL_{S1} ein und der invertierende Ausgang 180° koppelt das entsprechende Signal mit negativer Polarität in das rechte Segment WL_{S1b} des Sende-Wellenleiters WL_{S1} ein. Beide Signale laufen entlang der Segmente WL_{S1a}, WL_{S1b} des Wellenleiters WL_{S1} bis zu den Terminierungs-Widerständen R und koppeln während der Laufzeit in die Segmente WL_{E1a}, WL_{E1b} des Empfangs-Wellenleiters WL_{E1} über.

Der Abgriff A₁ am linken Empfangs-Wellenleiter befindet sich am Ende der Ausbreitungsstrecke des Eingangssignals. Empfangen wird ein invertiertes, hochpass-gefiltertes Signal. An den Stellen, an denen sich steile Signalflanken am Eingangssignal befanden, weist das Ausgangssignal somit schmale Pulse auf.

Der Abgriff A₂ am rechten Segment W_{LE1b} des Empfangs-Wellenleiters WL_{E1} befindet sich hingegen am Anfang der Ausbreitungsstrecke des Eingangssignals. Das Ausgangssignal besteht aus Pulsen, welche an den Stellen von Signalflanken des Eingangssignals auftreten, die Polarität des Signals bleibt jedoch unverändert. Die Pulsbreite ist größer als die des linken Segments WL_{E1a} des Empfangs-Wellenleiters WL_{E1} und entspricht der Summe der Laufzeiten des Eingangssignals entlang des rechten Segments WL_{S1b} des Sende-Wellenleiters WL_{S1} und des rechten WL_{S1b} Segments des Empfangs-Wellenleiters WL_{E1}.

Bei einer Verdrehung des Senders zum Empfänger um 180° werden die aus den Segmenten WL_{E1a}, WL_{E1b} des Empfangs-Wellenleiters WL_{E1} ausgekoppelten Signale vertauscht. Eine Verdrehung von ungleich 180° ergibt hingegen eine Überlagerung des langen und des kurzen Pulses an jedem der Segmente WL_{E1a}, WL_{E1b} des Empfangs-Wellenleiters WL_{E1}. Die beiden empfangenen Signale werden beispielsweise über einen resistiven 50/50-Koppler als Mittel ADD zur Addition aufaddiert. Das Ergebnis ist ein Signal, welches unabhängig vom Winkel der Verdrehung des Senders zum Empfänger eine hohe Stabilität aufweist. Das pulsförmige Signal wird anhand eines Mittels REC zur Signal-Rekonstruktion, beispielsweise in Form eines Integrators oder eines Komparators mit Hysterese, in das ursprüngliche rechteckförmige Signal rekonstruiert.

Figur 2 zeigt eine als Wellenleiter-Drehübertrager D ausgestaltete erfindungsgemäße Vorrichtung in symmetrischer Ausführung gemäß einem zweiten Ausführungsbeispiel. Das von der Signalquelle SQ ausgehende Eingangssignal wird dabei jeweils auf die invertierenden Eingänge 180° und die nichtinvertierenden Eingänge 0° zweier Komparatoren COMP₂, COMP₃ aufgeteilt.

Der Komparator COMP₃ koppelt beispielsweise das Signal in das linke Wellenleiterpaar, bestehend aus den Segmenten WL_{S1a}, WL_{S2a} der Sende-Wellenleiter WL_{S1} und WL_{S2}, ein. Der Komparator COMP₂ hingegen koppelt gemäß dem Ausführungsbeispiel das Signal in das rechte Wellenleiterpaar, bestehend aus den Segmenten WL_{S1b}, WL_{S2b} der Sende-Wellenleiters WL_{S1} und WL_{S2}, ein. Eine andere Beschaltung der Komparator-Ausgänge ist hierbei ebenfalls denkbar. Alternativ kann erfindungsgemäß auch der Einsatz eines Fan-Out-Buffers vorgesehen sein.

Auf der Empfängerseite werden die beiden an den äußeren Segmenten WL_{E2a}, WL_{E2b} der Wellenleiter empfangenen Signale durch ein Mittel ADD zur Addition addiert. Ferner werden in analoger Weise die beiden an den inneren Segmenten WL_{E1a}, WL_{E1b} der Wellenleiter empfangenen Signale ebenfalls durch ein Mittel ADD zur Addition addiert. Die Ergebnisse der beiden Additionen bilden ein symmetrisches Signal, aus dem der ursprüngliche rechteckförmige Datenstrom durch ein Mittel REC zur Signal-Rekonstruktion, vorliegend in Form eines Komparators mit Hysterese REC, wiederhergestellt wird.

## Patentansprüche

1. Vorrichtung zur Übertragung eines Signals mittels Wellenleitern in rotierenden Systemen, umfassend wenigstens einen ersten und einen zweiten Sende-Wellenleiter (WL_{S1}; WL_{S2}) und wenigstens einen Empfangs-Wellenleiter (WL_{E1}; WL_{E2}), wobei der wenigstens eine Empfangs-Wellenleiter (WL_{E1}; WL_{E2}) und der wenigstens erste und zweite Sende-Wellenleiter (WLs₁; WL_{S2}) in mehrere radial verteilte Segmente (WL_{E1a}, WL_{E1b}; WL_{E2a}, WL_{E2b}; WL_{S1a}, WL_{S1b}; WL_{S2a}, WL_{S2b}) unterteilt sind, wobei die radial verteilten Segmente (WL_{E1a}, WL_{E1b}; WL_{E2a}, WL_{E2b}) des wenigstens einen Empfangs-Wellenleiters (WL_{E1}; WL_{E2}) jeweils einen Abgriff (A_{1,} A₂) aufweisen und Mittel (ADD) zur Addition der mittels der Abgriffe (A_{1,} A₂) der radial verteilten Segmente (WL_{E1a}, WL_{E1b}; WL_{E2a}, WL_{E2b}) des wenigstens einen Empfangs-Wellenleiters (WL_{E1}; WL_{E2}) erhaltenen Signale vorgesehen sind, wobei Mittel (COMP₂, COMP₃) zur Aufteilung des zu übertragenden Signals für den ersten Sende-Wellenleiter (WL_{S1}) und für den zweiten Sende-Wellenleiter (WL_{S2}) vorgesehen sind.

2. Vorrichtung nach Anspruch 1, wobei sich die Abgriffe (A_{1,} A₂) der radial verteilten Segmente (WL_{E1a}, WL_{E1b}; WL_{E2a}, WL_{E2b}) des wenigstens einen Empfangs-Wellenleiters (WL_{E1}; WL_{E2}) gegenüberliegen.

3. Vorrichtung nach Anspruch 1 oder Anspruch 2, ferner umfassend Mittel zur Einkopplung des zu übertragenden Signals mit positiver Polarität in ein erstes Segment (WL_{S1}) des wenigstens einen Sende-Wellenleiters (WLs₁; WL_{S2}) und zur Einkopplung des zu übertragenden Signals mit negativer Polarität in ein zweites Segment (WL_{S2}) des wenigstens einen Sende-Wellenleiters (WLs₁; WL_{S2}).

4. Vorrichtung nach Anspruch 3, wobei als Mittel zur Einkopplung des zu übertragenden Signals ein Komparator (COMP₁; COMP₂, COMP₃) vorgesehen ist, welcher einen nichtinvertierenden (0°) und einen invertierenden (180°) Ausgang aufweist.

5. Vorrichtung nach einem der Ansprüche 1 bis 4, wobei zur Terminierung eines freibleibenden Endes der radial verteilten Segmente des wenigstens einen Sende-Wellenleiters (WLs₁; WL_{S2}) und/oder des wenigstens einen Empfangs-Wellenleiters (WL_{E1}, WL_{E2}) jeweils ein Widerstand (R) vorgesehen ist.

6. Vorrichtung nach einem der Ansprüche 1 bis 5, wobei als Mittel (ADD) zur Addition ein resistiver Koppler vorgesehen ist.

7. Vorrichtung nach einem der Ansprüche 1 bis 6, ferner umfassend Mittel (REC) zur Rekonstruktion des durch Addition erhaltenen Signals.

8. Vorrichtung nach Anspruch 7, wobei als Mittel (REC) zur Rekonstruktion ein Integrator oder ein Komparator mit Hysterese vorgesehen ist.

9. Vorrichtung nach einem der Ansprüche 1 bis 8, wobei als Segmente für den wenigstens einen Sende-Wellenleiter (WLs₁; WL_{S2}) und/oder für den wenigstens einen Empfangs-Wellenleiter (WL_{E1}; WL_{E2}) Halbringe vorgesehen sind.

10. Vorrichtung nach einem der Ansprüche 1 bis 8, wobei als Segmente für den wenigstens einen Sende-Wellenleiter (WLs₁; WL_{S2}) und/oder für den wenigstens einen Empfangs-Wellenleiter (WL_{E1}; WL_{E2}) verkürzte Empfangsringe mit gegenüberliegenden Auskoppelpunkten vorgesehen sind.

11. Vorrichtung nach einem der Ansprüche 1 bis 10, wobei die Länge der Segmente des wenigstens einen Empfangs-Wellenleiters (WL_{E1}; WL_{E2}) größer ist als die minimal im zu übertragenden Signal vorkommende relevante Wellenlänge *λ*ₘᵢₙ= *c*2π*τ*.

12. Vorrichtung nach einem der Ansprüche 1 bis 11, wobei der wenigstens eine Sende-Wellenleiter (WLs₁; WL_{S2}) an einem Rotor und der wenigstens eine Empfangs-Wellenleiter (WL_{E1}; WL_{E2}) an einem Stator vorgesehen sind.

13. Vorrichtung nach Anspruch 1, ferner umfassend einen zweiten Empfangs-Wellenleiter (WL_{E2}), wobei der erste Empfangs-Wellenleiter (WL_{E1}) zur Erfassung des mittels des ersten Sende-Wellenleiters (WL_{S1}) übertragenen Signals und der zweite Empfangs-Wellenleiter (WL_{E2}) zur Erfassung des mittels des zweiten Sende-Wellenleiters (WL_{S2}) übertragenen Signals vorgesehen sind.

## Claims

1. Apparatus for transmitting a signal by means of waveguides in rotating systems, comprising at least one first and one second transmitting waveguide (WL_{S1}; WL_{S2}) and at least one receiving waveguide (WL_{E1}; WL_{E2}), wherein the at least one receiving waveguide (WL_{E1}; WL_{E2}) and the at least first and second transmitting waveguides (WL_{S1}; WL_{S2}) are subdivided into a plurality of radially distributed segments (WL_{E1a} WL_{E1b}; WL_{E2a}, WL_{E2b}; WL_{S1a}, WL_{S1b}; WL_{S2a}, WL_{S2b}), wherein the radially distributed segments (WL_{E1a} WL_{E1b}; WL_{E2a}, WL_{E2b}) of the at least one receiving waveguide (WL_{E1}; WL_{E2}) each have a tap (A₁, A₂), and means (ADD) for adding the signals received by means of the taps (A₁, A₂) of the radially distributed segments (WL_{E1a} WL_{E1b}; WL_{E2a}, WL_{E2b}) of the at least one receiving waveguide (WL_{E1}; WL_{E2})are provided, wherein means (COMP₂, COMP₃) for splitting the signal to be transmitted for the first transmitting waveguide (WL_{S1}) and for the second transmitting waveguide (WL_{S2}) are provided.

2. Apparatus according to Claim 1, wherein the taps (A₁, A₂) of the radially distributed segments (WL_{E1a}, WL_{E1b}; WL_{E2a}, WL_{E2b}) of the at least one receiving waveguide (WL_{E1}; WL_{E2})are opposite one another.

3. Apparatus according to Claim 1 or Claim 2, also comprising means for coupling the signal to be transmitted with positive polarity into a first segment (WL_{S1}) of the at least one transmitting waveguide (WL_{S1}; WL_{S2}) and for coupling the signal to be transmitted with negative polarity into a second segment (WL_{S2})of the at least one transmitting waveguide (WL_{S1}; WL_{S2}) .

4. Apparatus according to Claim 3, wherein a comparator (COMP₁; COMP₂, COMP₃) having a non-inverting output (0°) and an inverting output (180°) is provided as the means for coupling in the signal to be transmitted.

5. Apparatus according to one of Claims 1 to 4, wherein a resistor (R) is respectively provided for the purpose of terminating a free end of the radially distributed segments of the at least one transmitting waveguide (WL_{S1}; WL_{S2}) and/or of the at least one receiving waveguide (WL_{E1}; WL_{E2}).

6. Apparatus according to one of Claims 1 to 5, wherein a resistive coupler is provided as the adding means (ADD).

7. Apparatus according to one of Claims 1 to 6, also comprising means (REC) for reconstructing the signal obtained by means of addition.

8. Apparatus according to Claim 7, wherein an integrator or a comparator with hysteresis is provided as the reconstruction means (REC).

9. Apparatus according to one of Claims 1 to 8, wherein half-rings are provided as segments for the at least one transmitting waveguide (WL_{S1}; WL_{S2}) and/or for the at least one receiving waveguide (WL_{E1}; WL_{E2}).

10. Apparatus according to one of Claims 1 to 8, wherein shortened receiving rings having opposite coupling-out points are provided as segments for the at least one transmitting waveguide (WL_{S1}; WL_{S2}) and/or for the at least one receiving waveguide (WL_{E1}; WL_{E2}).

11. Apparatus according to one of Claims 1 to 10, wherein the length of the segments of the at least one receiving waveguide (WL_{E1}; WL_{E2})is greater than the minimum relevant wavelength λₘᵢₙ=c2πτ that occurs in the signal to be transmitted.

12. Apparatus according to one of Claims 1 to 11, wherein the at least one transmitting waveguide (WL_{S1}; WL_{S2}) is provided on a rotor and the at least one receiving waveguide (WL_{E1}; WL_{E2}) is provided on a stator.

13. Apparatus according to Claim 1, also comprising a second receiving waveguide (WL_{E2}), wherein the first receiving waveguide (WL_{E1}) is provided for the purpose of capturing the signal transmitted by means of the first transmitting waveguide (WL_{S1}) and the second receiving waveguide (WL_{E2}) is provided for the purpose of capturing the signal transmitted by means of the second transmitting waveguide (WL_{S2}).

## Revendications

1. Dispositif permettant de transmettre un signal au moyen de guides d'ondes dans des systèmes rotatifs, comprenant au moins un premier et un deuxième guide d'ondes d'émission (WL_{S1} ; WL_{S2}) et au moins un guide d'ondes de réception (WL_{E1} ; WL_{E2}), ledit au moins un guide d'ondes de réception (WL_{E1} ; WL_{E2}) et les au moins premier et deuxième guides d'ondes d'émission (WL_{S1} ; WL_{S2}) sont divisés en plusieurs segments répartis radialement (WL_{E1a}, WL_{E1b} ; WL_{E2a}, WL_{E2b} ; WL_{S1a}, WL_{S1b} ; WL_{S2a}, WL_{S2b}),
dans lequel les segments répartis radialement (WL_{E1a}, WL_{E1b} ; WL_{E2a}, WL_{E2b}) du au moins un guide d'ondes de réception (WL_{E1} ; WL_{E2}) présentent respectivement une prise (A₁, A₂), et des moyens (ADD) permettant d'additionner les signaux obtenus au moyen des prises (A₁, A₂) des segments répartis radialement (WL_{E1a}, WL_{E1b} ; WL_{E2a}, WL_{E2b}) du au moins un guide d'ondes de réception (WL_{E1} ; WL_{E2})sont prévus, des moyens (COMP₂, COMP₃) étant prévus pour diviser le signal à transmettre pour le premier guide d'ondes d'émission (WL_{S1}) et pour le deuxième guide d'ondes d'émission (WL_{S2}).

2. Dispositif selon la revendication 1, dans lequel les prises (A₁, A₂) des segments répartis radialement (WL_{E1a}, WL_{E1b} ; WL_{E2a}, WL_{E2b}) du au moins un guide d'ondes de réception (WL_{E1} ; WL_{E2})sont opposées.

3. Dispositif selon la revendication 1 ou 2, comprenant en outre des moyens pour le couplage du signal à transmettre à polarité positive dans un premier segment (WL_{S1}) du au moins un guide d'ondes d'émission (WL_{S1} ; WL_{S2}) et pour le couplage du signal à transmettre à polarité négative dans un deuxième segment (WL_{S2}) du au moins un guide d'ondes d'émission (WL_{S1} ; WL_{S2}).

4. Dispositif selon la revendication 3, dans lequel, comme moyen de couplage du signal à transmettre, un comparateur (COMP₁ ; COMP₂, COMP₃) est prévu qui présente une sortie non inverseuse (0°) et une sortie inverseuse (180°).

5. Dispositif selon l'une quelconque des revendications 1 à 4, dans lequel respectivement une résistance (R) est prévue pour terminer une extrémité restant libre des segments répartis radialement du au moins un guide d'ondes d'émission (WL_{S1} ; WL_{S2}) et/ou du au moins un guide d'ondes de réception (WL_{E1}, WL_{E2}).

6. Dispositif selon l'une quelconque des revendications 1 à 5, dans lequel un coupleur résistif est prévu comme moyen (ADD) d'addition.

7. Dispositif selon l'une quelconque des revendications 1 à 6, comprenant en outre des moyens (REC) permettant de restituer le signal obtenu par addition.

8. Dispositif selon la revendication 7, dans lequel un intégrateur ou un comparateur à hystérésis est prévu comme moyen (REC) de restitution.

9. Dispositif selon l'une quelconque des revendications 1 à 8, dans lequel des demi-anneaux sont prévus comme segments pour ledit au moins un guide d'ondes d'émission (WL_{S1} ; WL_{S2}) et/ou pour ledit au moins un guide d'ondes de réception (WL_{E1} ; WL_{E2}).

10. Dispositif selon l'une quelconque des revendications 1 à 8, dans lequel des anneaux de réception raccourcis à points de découplage opposés sont prévus comme segments pour ledit au moins un guide d'ondes d'émission (WL_{S1} ; WL_{S2}) et/ou pour ledit au moins un guide d'ondes de réception (WL_{E1} ; WL_{E2}).

11. Dispositif selon l'une quelconque des revendications 1 à 10, dans lequel la longueur des segments du au moins un guide d'ondes de réception (WL_{E1} ; WL_{E2})est supérieure à la longueur d'onde pertinente λₘᵢₙ = c2πτ apparaissant dans le signal à transmettre.

12. Dispositif selon l'une quelconque des revendications 1 à 11, dans lequel ledit au moins un guide d'ondes d'émission (WL_{S1} ; WL_{S2}) est prévu sur un rotor, et ledit au moins un guide d'ondes de réception (WL_{E1} ; WL_{E2}) est prévu sur un stator.

13. Dispositif selon la revendication 1, comprenant en outre un deuxième guide d'ondes de réception (WL_{E2}), le premier guide d'ondes de réception (WL_{E1}) étant prévu pour détecter le signal transmis au moyen du premier guide d'ondes d'émission (WL_{S1}), et le deuxième guide d'ondes de réception (WL_{E2}) étant prévu pour détecter le signal transmis au moyen du deuxième guide d'ondes d'émission2 (WL_{S2}).
